# EUROPEAN PATENT APPLICATION

(11) **EP 3 338 760 A1**
(43) Date of publication of application: **27.06.2018**
(21) Application number: 18155990.7
(22) Date of filing: 31.08.2010
(51) Int. Cl.: A61K 8/365, A61K 8/60, A61K 8/67, A61Q 19/08

(54) **COSMETIC COMPOSITION**

(30) Priority: 11.09.2009 GB 0915964
(62) Divisional of application: 10751711.2
(71) Applicant: Reckitt Benckiser Healthcare International Limited, Slough, Berkshire SL1 3UH (GB)
(72) Inventor: BUCKLEY, Carolyn, Hull, Humberside HU8 7DS (GB); JACKSON, Stuart, Hull, Humberside HU8 7DS (GB); RAWLINGS, Anthony, Kingsmead, Cheshire CW9 8FH (GB)
(74) Representative: Bowers, Craig Malcolm

(57) **Abstract**

A dermatological or cosmetic composition for skin care which includes a salt of an ahydroxy acid, beta -hydroxy acid, poly-hydroxy acid or bionic acid selected from one or more of gluconic acid, glycolic acid, lactic acid, salicylic acid, mandelic acid, benzilic acid, napthoic acid, malic acid, tartaric acid, lactobionic acid and maltobionic acid; in combination with niacinamide present in an amount of more than 0.5 percent by weight, and less than 5 percent by weight.

## Description

The present invention is concerned with improved dermatological and cosmetic compositions, particularly for mature skin having improved skin benefits, in particular improved skin barrier.

It is therefore an object of the present invention to provide improved dermatological and cosmetic compositions. It is another object of the invention to provide a novel method for improving skin barrier, particularly for mature skin where a number effects from aging have a negative effect on the overall skin barrier. It is particularly preferred object of the present invention to provide a composition for hydrating skin, which will improve skin physiology, texture, or health, especially in mature skin. These and other objects and advantages of the invention will become obvious from the following detailed description.

Accordingly, there is provided a dermatological or cosmetic composition for skin care which includes a salt of an α-hydroxy acid, β-hydroxy acid, poly-hydroxy acid or bionic acid selected from one or more of gluconic acid, glycolic acid, lactic acid, salicylic acid, mandelic acid, benzilic acid, napthoic acid, malic acid, tartaric acid, lactobionic acid and maltobionic acid; in combination with niacinamide.

Niacinamide, also known as nicotinamide, is the physiologically active amide derivative of nicotinic acid (also known as niacin and vitamin B3). It is a water-soluble vitamin abundant in meat, yeast, fruits and many vegetables. Niacinamide has a number of well documented benefits on the skin barrier such as
- replenishing niacinamide coenzymes which deplete with increasing age in skin
- stimulating aged fibroblasts to stimulate collagen synthesis
- improving skin barrier function by up-regulating epidermal ceramides synthesis
- improving keratinocyte differentiation which slows with increasing age
- preventing UV damage to skin and inhibiting UV immune suppression
- inhibiting melanosome transfer from melanocytes to keratinocytes, leading to a reduction in skin pigmentation with time
- reducing sebaceous activity in facial skin
- improving skin smoothness
- improving wound healing.

The main function of hydroxy-acids is to improve skin turnover and some, such as gluconic acid, have additional benefits such as
- good skin tolerability
- moisturising
- don't thin strateum corneum, with no increase in sun sensitivity
- strengthen skin barrier
- free radical scavenging effect
- improve appearance of photodamaged skin.

Improving skin turnover helps skin repair, it provides a more efficient skin barrier and healthier cells in the epidermis, and as a result the skin looks and feels smoother.

If hydroxy acids improve skin turnover and the health of epidermis, niacinamide helps to improve the structure of the skin - lipids in barrier, moisturisation, collagen in dermis. The composition of the present invention addresses the deficits in mature skin and acts synergistically to make skin stronger and more resistant to stresses e.g. low humidity, cold (which is clinically tested by a stress test).

It is believed that the combination of niacinamide and an acid salt provides a synergistic effect compared to the use of either alone; the resultant composition has enhanced skin barrier on a user's skin.

The acid salt is preferably selected from sodium, potassium, ammonium, amine salts such as triethanolamine and arginine, zinc, copper and magnesium.

Further preferably, the acid salt is a gluconate salt. In a further preferred embodiment, the composition comprises two acid salts, preferably gluconate and salicylate.

According to a particularly preferred embodiment of the present invention, the acid salt and the niacinamide are present in the composition in a combined amount in the range 0.1 to 20% by weight of the formulation, typically in the range 1.0% to 15% by weight of the composition.

The acid salt is preferably present in an amount of at least 0.1% by weight of the composition, further preferably at least 2.0% by weight of the composition.

Typically the acid salt is not present in an amount greater than 10% by weight of the composition, typically no greater than 7% of the composition. Desirably, the acid salt is present in the range 3.5% to 6.5%. A particularly preferred amount of the acid salt is about 5% by weight of the composition.

The concentration of niacinamide in the composition is at least 0.01 percent by weight, more preferably at least 0.1 percent by weight and most preferably at least 0.5 percent by weight, and is less than 10 percent by weight, more preferably less than 5 percent by weight.

Preferably, the compositions are characterized in that they are presented in the form of liquid or solid preparations for topical use and may be preferably presented in one of the following forms:
fatty gels, simple water-in-oil emulsions, simple oil-in-water emulsions, multiple emulsions, for example: water-in-oil-in-water or oil-in-water-in-oil, a triple water-in-oil-in-water emulsion, a triple oil-in-water-in-oil emulsion, an oil-in-water emulsion containing liquid crystals, complex emulsions containing liquid crystals forming lipid double layers surrounding the oily phases, pseudo-emulsions (dispersion of an oily phase or a water-in-oil emulsion in a gelatinized aqueous phase, without traditional surfactants), oil-in-water or water-in-oil micro-emulsions, emulsions containing dispersed oil phases, different from each other and insoluble in each other, a pseudo-emulsion or dispersion of an oily phase dispersed in an aqueous phase and stabilized with Lubragel, Pemulen, Hypan, xanthan gum, CMC, hydroxyethyl cellulose, Amigel, Polyvinyl-pyrrolidone, Amercell HM1500, or a mixture of two or more of these gelatinizing agents.

The composition according to the invention may be formulated in numerous forms; however, the composition may often take the form of an aqueous or oily solution or surfactant wash or dispersion or emulsion or a gel

Gels provided according to the invention may be aqueous or non-aqueous. Aqueous gels are preferred. The gel will contain a gelling agent in order to give sufficient viscosity to the gel

It is envisaged that the formulations according to the present invention may be 'leave-on' composition (that is composition applied to the skin and subsequently left on the skin) or 'wash-off composition (that is compositions applied to the skin and subsequently rinsed away, by for example water, shortly after application).

In the case of solutions or dispersions, and gels, the composition will generally contain a solvent system or other continuous liquid phase. Such a system is preferably aqueous; however, mixed solvent systems may often be used with advantage. Such a mixed solvent system most preferably comprises water, in admixture with a co-solvent, most preferably a lower (eg C₁₋₆) alcohol, in particular ethanol and t-butyl alcohol.

Preferred aqueous systems comprise water in an amount of at least 25 percent by weight, more preferably at least 35 percent by weight, The upper limit of water will depend on the amounts of other ingredients incorporated in the composition so that the water may form the remainder of the composition up to 100 percent of the composition.

The composition may additionally comprise other components which will be well known to those skilled in the art. These include, for example:

### (i) Emulsifiers

Any emulsifiers known in the art for use in water-in-oil or oil-in-water emulsions or microemulsions, and may be included alone or in combination with another emulsion. Known cosmetically acceptable emulsifiers may include: a) sesquioleates such as sorbitan sesquioleate, available commercially for example under the trade name Arlacel 83 (ICI), or polyglyceryl-2- sesquioleate; b) ethoxylated esters of derivatives of natural oils such as the polyethoxylated ester of hydrogenated castor oil available commercially for example under the trade name Arlacel 989 (ICI); c) silicone emulsifiers such as silicone polyols available commercially for example under the trade name ABIL WS08 (Th. Goldschmidt AG); d) anionic emulsifiers such as fatty acid soaps e.g. potassium stearate and fatty acid sulphates e.g. sodium cetostearyl sulphate available commercially under the trade name Dehydag (Henkel); e) ethoxylated fatty alcohols, for example the emulsifiers available commercially under the trade name Brij (ICI); f) sorbitan esters, for example the emulsifiers available commercially under the trade name Span (ICI); g) ethoxylated sorbitan esters, for example the emulsifiers available commercially under the trade name Tween (ICI); h) ethoxylated fatty acid esters such as ethoxylated stearates, glyceryl monostearates for example the emulsifiers available commercially under the trade name Myrj (ICI); i) ethoxylated mono-, di-, and tri-glycerides, for example the emulsifiers available commercially under the trade name Labrafil (Alfa Chem.); j) non-ionic self-emulsifying waxes, for example the wax available commercially under the trade name Polawax (Croda); k) ethoxylated fatty acids, for example, the emulsifiers available commercially under the trade name Tefose (Alfa Chem.) l) methylglucose esters such as polyglycerol-3 methyl glucose distearate available commercially under the name Tegocare 450 (Degussa Goldschmidt);. m) as well as polyacrylamide emulsifier systems for examples cream gel emulsifier under trade name Sepigel 305 (Seppic), n) polymeric emulsifiers for example, the emulsifiers available commercially under the trade name Permulen, o) glyceryl stearate for example, the emulsifiers available commercially under the trade name Tegin, p) cationic emulsifiers, q) alkyl polyglyceride or p) mixtures thereof.

### (ii) Emollients

These are ingredients that help to maintain the soft, smooth and pliable appearance of skin. Such ingredients may function by their ability to remain on the surface of the skin or in the stratum corneum, and to act as lubricants, reducing or preventing flaking of the skin and improving the skin's appearance. Examples of emollients are,but are not exclusive to: hydrocarbon oils such as paraffin or mineral oils; b) waxes such as beeswax or paraffin wax; c) natural oils such as sunflower oil, apricot kernel oil, shea butter or jojoba oil; d) silicone oils such as dimethicone, cyclomethicone or cetyldimethicone; e) fatty acid esters such as isopropyl palmitate, isopropyl myristate, dioctylmaleate, glyceryl oleate and cetostearyl isononanoate; f) fatty alcohols such as cetyl alcohol or stearyl alcohol and mixtures thereof (eg cetearyl alcohol); g) polypropylene glycol or polyethylene glycol ethers, eg PPG-14 butyl ether; or h) mixtures thereof, for example, the blend of waxes available commercially under the trade name Cutina (Henkel) i) triglycerides of fatty acids eg lauric triglyceride or capric/caprylic triglyceride, such as the triglyceride available commercially under the trade name Miglyol 810 (Huls UK) or j) alcohol emollients such as octyldodecanol, k) fatty alcohols and I) fatty acids.

### (iii) Humectants or Moisturisers

These are ingredients intended to increase the water content of the top layers of the skin. Examples of such ingredients are glycerin, sorbitol, sorbitol, hydroxyethyl urea, 1,3-butylene glycol and propylene glycol.

### (iv) Thickening Agents/Gelling Agents

A variety of thickening agents may also be used according to the nature of the liquid carrier and the viscosity required. Thickeners that are water-soluble or hydrophilic are preferred, and examples include acrylic acid polymers, eg those available commercially under the trade name Carbopol (B.F. Goodrich), modified celluloses, eg hydroxypropylmethylcellulose or hydroxyethylcellulose available commercially under the trade name Natrosol (Hercules), alkylgalactomanans available under the trade name N-Hance, xanthan gum, magnesium aluminium silicate and sodium chloride.

The amount of gelling and/or thickening agent in the composition will each preferably lie in the range 0.01 to 3 percent w/w, more preferably 0.05 to 2.0 percent w/w. Typically, the amount of gelling and/or thickening agent will each be less than 1 percent w/w, eg about 0.1 percent w/w or about 0.8 percent w/w.

### (v) Surfactants

Surfactants may be used in compositions according to the invention as solubilisers, or as cleansing agents or foam boosters. Many different classes of surfactant may be suitable for inclusion in the composition according to the invention, and these will be readily apparent to those skilled in the art. Examples of suitable surfactants include anionic surfactants (eg sodium laureth sulphate, non ionic surfactants (eg cocoglucoside) cationic surfactants and/or amphoteric surfactants (eg cocoamidoproyl betaine).Polyethylene glycol ethers of alcohols such as isocetyl alcohol (eg Isoceteth- 20), isostearyl alcohol (eg Isosteareth-20), cetyl alcohol (eg Ceteth-20), oleyl alcohol (eg Oleth-20) and cetearyl alcohol (eg Ceteareth-20).

### (vi) Preservatives

These are ingredients which prevent or retard microbial growth and thus protect the composition from spoilage. Examples of preservatives include such as phenoxyethanol, methyl paraben, propylparaben, bronopol, sodium dehydroacetate, polyhexamethylenebiguanide hydrochloride, isothiazolone, ethyl hexyl glycerin, sorbic acid, dehydroacetic acid, Polyamidopropyl Biguanide and diazolidinylurea.

### (vii) Chelating agents or sequestering agents (sequestrants)

These are ingredients that have the ability to complex with and inactivate metallic ions in order to prevent their adverse effects on the stability or appearance of the composition, as described above. Examples of chelating agents are ethylenediamine tetraacetic acid and its salts, notably the dipotassium and especially the disodium or tetrasodium salt.

### (viii) Opacifying agents

For example, agents such as clays (e.g. kaolin and bentonite) as well as titanium dioxide.

### (ix) pH adjusters

These are ingredients used to control the pH of the composition. Examples of pH adjusters are inorganic salts such as sodium hydroxide, and organic bases such as triethanolamine and arginine.

### (x) Conditioning agents, for example distearyldimonium chloride.

### (xi) Perfumes and colourings.

The compositions of the invention can optionally also contain hydrosoluble or liposoluble active ingredients, filters, screens for solar radiation, vitamin extracts, anti-oxidants, antimicrobials, antibacterials, physical exfoliating agents or, coloring agents.

The composition according to the invention preferably has a viscosity of from about 1,000 mPa.s to about 200,000 mPa.s, Viscosity may be measured using a Brookfield RVT viscometer equipped with a T bar B rotating at 20rpm for 1 minute.

The different cosmetic or dermatological forms mentioned above can be obtained according to the usual methods used in this domain. The dermatological and cosmetic compositions can be solid or liquid or in paste form and be presented in the dermatological and cosmetic forms commonly used such as creams or gels in pots or in tubes, lotions in glass or plastic bottles and optionally in dropping bottles, phials, fluids, ointments. They are prepared according to the usual methods.

According to a further aspect of the present invention, there is provided a method of restoring the barrier function of the stratum corneum of skin comprising applying to said skin an effective amount of the composition of the present invention.

According to a yet further aspect of the present invention, there is provided a method of manufacture of a composition as hereinbefore described which comprises the steps of:
- preparation of an oil phase
- preparation of an aqueous phase
- blending of the oil and aqueous phases to give a homogeneous emulsion
- addition of nicotinamide to the emulsion followed by further homogenisation
- addition of the acid salt followed by further homogenisation.

The invention will now be described in greater detail, by way of illustration only, with reference to the following Examples 1 to 6. The composition of each of these examples is shown fully in the following Table 1 where the amounts are shown in wt%.

**TABLE 1**

| | | 1 | 2 | 3 | 4 | 5 | 6 |
|---|---|---|---|---|---|---|---|
| Water | Solvent | 64.408 | 67.298 | 65.308 | 64.908 | 65.308 | 63.708 |
| Glycerin | humectant | 12 | 12 | 12 | 12 | 12 | 12 |
| Liquid paraffin | emollient | 7.5 | 6 | 7.5 | 7.5 | 7.5 | 7.5 |
| Sodium Gluconate | humectant | 5 | 5 | 5 | 5 | 5 | 5 |
| Glyceryl Stearate | emulsifier | 3.5 | 2.5 | 0 | 0 | 0 | 0 |
| Dimethicone | silicone | 2.5 | 2 | 2.5 | 2.5 | 2.5 | 2,7 |
| Nicotinamide | Active | 2 | 2 | 2 | 2 | 2 | 2 |
| Salicylic acid | Active | 0 | 0 | 0 | 0 | 0 | 1 |
| Rice Starch | conditioner | 1.5 | 1.5 | 1.5 | 1.5 | 1.5 | 1.5 |
| Phenoxyethanol | preservative | 0.5 | 0 | 0.5 | 0.5 | 0.5 | 0.5 |
| Sodium Cetearyl Sulfate | emulsifier | 0.3 | 0.25 | 0 | 0 | 0 | 0 |
| Xanthan Gum | thickener | 0.15 | 0.15 | 0.1 | 0.15 | 0.1 | 0.1 |
| Veegum Ultra | thickener | 0.15 | 0.15 | 0.1 | 0.15 | 0.1 | 0.1 |
| Methyl Paraben | preservative | 0.15 | | 0.15 | 0.15 | 0.15 | 0.15 |
| Fragrance | fragrance | 0.1 | 0.1 | 0.1 | 0.1 | 0.1 | 0.1 |
| Tocopheryl Acetate | antioxidant | 0.1 | 0.1 | 0.1 | 0.1 | 0.1 | 0.1 |
| Sunflower Oil | | 0.05 | 0.05 | 0.05 | 0.05 | 0.05 | 0.05 |
| Almond Oil | | 0.05 | 0.05 | 0.05 | 0.05 | 0.05 | 0.05 |
| Propyl Paraben | preservative | 0.04 | | 0.04 | 0.04 | 0.04 | 0.04 |
| Polyquaternium-51 | Active | 0.002 | 0.002 | 0.002 | 0.002 | 0.002 | 0.002 |
| Phenoxyethanol, Benzoic Acid, Dehydroacetic Acid, Polyamidopropyl Biguanide, Aqua, Ethylhexyl-glycerin | Preservative mixture | 0 | 0.6 | 0 | 0 | 0 | 0 |
| Carbomer | thickener | 0 | 0.25 | 0 | 0.3 | 0 | 0 |
| Cetearyl Alcohol (and) Glyceryl Stearate (and) PEG-40 Stearate (and) Ceteareth-20 | Emulsifier blend | | | 3 | | | |
| Steareth-2 | emulsifier | 0 | 0 | 0 | 2 | 0 | 0 |
| Steareth-21 | emulsifier | 0 | 0 | 0 | 1 | 0 | 0 |
| 30% NaOH solution | pH adjust | 0 | 0 | 0 | 0 | 0 | 0.6 |
| | | 100 | 100 | 100 | 100 | 100 | 100 |

### Method

### 1. Oil Phase

The oil phase is prepared in a clean, appropriately sized vessel by adding Pionier 2076, Silicon Oil, Tocopheryl Acetate and Lipomulse to the vessel and mixing until clear, following which the oil phase is heated to 70°C.

### 2. Aqueous Phase

The aqueous phase is prepared by firstly cleaning an appropriately sized vessel to use as the main vessel and adding deionised water and the Xanthan Gum and Veegum Ultra to the main vessel and mix with an overhead stirrer until dissolved, followed by warming to 70-75°C. Then the glycerin is added and stirred at low speed, followed by the methylparaben and propylparaben and the mixture is stirred until uniform.

### 3. Phase Combination

The oil phase is added very slowly to the main vessel, followed by the Almond and Sunflower Oils, and the phases are combined with strong homogenisation for approximately 2-3 minutes to give a fully homogeneous mixture. Then the nicotinamide is added and homogenised until fully dispersed. The mixture is coooled to 35°C with slow stirring.

### 4. Remaining Ingredients

The sodium gluconate solution is prepared and added, and the mixture is homogenized slowly until uniform. The contents of the main vessel are cooled to 30°C, and once this temperature has been reached, phenoxyethanol, Lipidure and Ombrosia are added and stirred at low speed. If needed, for instance, when a second acid such as salicylic acid is used (Example 6), the pH is adjusted to 5.2 by adding the sodium hydroxide solution and stirring at low speed to ensure good distribution of the sodium hydroxide. Following this, the vessel is emptied and the next day the pH and viscosity are checked.

## Claims

1. A dermatological or cosmetic composition for skin care which includes a salt of an a-hydroxy acid, beta -hydroxy acid, poly-hydroxy acid or bionic acid selected from one or more of gluconic acid, glycolic acid, lactic acid, salicylic acid, mandelic acid, benzilic acid, napthoic acid, malic acid, tartaric acid, lactobionic acid and maltobionic acid; in combination with niacinamide present in an amount of more than 0.5 percent by weight, and less than 5 percent by weight.

2. A composition according to claim 1, wherein the acid salt is selected from from sodium, potassium, ammonium, triethanolamine, arginine, zinc, copper and magnesium salts.

3. A composition according to either claim 1 or claim 2, wherein the acid salt and the niacinamide are present in the composition in a combined amount in the range 0.1 to 20 percent by weight of the formulation, typically in the range 1.0 percent to 15 percent by weight of the composition.

4. A composition according to any preceding claim, wherein the acid salt is present in an amount of at least 0.1 percent by weight of the composition.

5. A composition according to claim 4, wherein the acid salt is present in an amount of at least 2.0 percent by weight of the composition.

6. A composition according to any preceeding claim, wherein the acid salt is present in an amount no greater than 10 percent by weight of the composition.

7. A composition according to claim 6, wherein the acid salt is present in an amount no greater than 7 percent by weight of the composition.

8. A composition according to any preceding claim, wherein the acid salt is present in the range 3.5 percent to 6.5 percent.

9. A composition according to any preceding claim wherein the acid salt is a gluconate salt.

10. A composition according to any preceding claim which comprises two acid salts. 1 1.

11. A composition according to claim 10 wherein the two acid salts are gluconate and salicylate.

12. A composition according to any preceding claim which is in the form of liquid or solid preparations for topical use.

13. A composition according to any preceding claim, which is in the form of an aqueous, oily solution, surfactant wash, dispersion, emulsion or gel.

14. A method of restoring the barrier function of the stratum corneum of skin comprising applying to said skin an effective amount of the composition according to any of claims 1 to 11.

15. A method of manufacture of a composition according to any of claims 1 to 13 which comprises the steps of:
preparation of an oil phase;
preparation of an aqueous phase;
blending of the oil and aqueous phases to give a homogeneous emulsion;
addition of nicotinamide to the emulsion followed by further homogenisation; and,
addition of the acid salt followed by further homogenisation.
